# EUROPEAN PATENT APPLICATION

(11) **EP 4 282 447 A1**
(43) Date of publication of application: **29.11.2023**
(21) Application number: 22175846.9
(22) Date of filing: 27.05.2022
(51) Int. Cl.: A61L 27/38, A61L 27/44, A61L 27/56, A61L 27/54, A61L 27/26

(54) **BIOACTIVE THREE-DIMENSIONAL CELLULAR CONSTRUCT FOR REGENERATION OF SYMPTOMIC, LOCAL CARTILAGE DEFECTS**

(71) Applicant: Lietuvos sveikatos mokslu universitetas, 44307 Kaunas (LT)
(72) Inventor: Ma iulaitis, Justinas, LT-44307 Kaunas (LT); Ma iulaitis, Romaldas, LT-44307 Kaunas (LT); Gudas, Rimtautas, Kaunas (LT)
(74) Representative: Pakeniene, Ausra

(57) **Abstract**

A three-dimensional scaffold comprised of polycaprolactone (poly-hexane-6-lactone), PLGA (poly-D,L-lactide-co-glycolide) and magnesium mydroxide ((Mg(OH)₂). Polycaprolactone is a biodegradable and biocompatible polyester resistant to mechanical impact. PLGA was selected with a lactic acid monomer content of 75% in the polymer and a glycolic acid monomer content of 25%. H₂MgO₂ particles were selected as functional agents allowing the neutralization of the acidic degradation product of polymer composites. The scaffold comprises antacid and antiinflammatory properties exhibited *in vitro.* Incorporated magnesium hydroxide in biodegradable polymer scaffolds demonstrated and effective inhibition of the acidification by polymer degradation and improved the cell viability during chondrogenic proliferation. In addition, magnesium hydroxide could also inhibit inflammatory responses caused by the acidic degradation of the scaffold.

## Description

### TECHNICAL FIELD

The invention relates to bioactive three-dimensional construct for use for cartilage regeneration.

### BACKGROUND ART

Articular cartilage (AC) defects remain a significant problem, especially for population with a high level of physical activity. Incidental finding of chondral lesions in 63% of knee arthroscopic surgeries, present a big challenge to orthopedic surgeons (Curl et al). Single localized defects have been previously treated with established surgical techniques such as microfracture (MF), osteochondral graft transplantation (OAT), autologous chondrocyte transplantation (ACI), autologous matrix-induced chondrogenesis (AMIC) and matrix induced autologous chondrocyte implantation (MACI). Curl et al., however, concluded that people who lead an active lifestyle in sports have an increased risk for acquiring larger and often multiple lesions, which require personalized treatment approaches and have a higher risk of early osteoarthritis development. In addition, a tendency toward more than one lesion (multiple complex lesions) was found during knee arthroscopic surgery in a multitude of cases. The treatment method is usually based on patient's clinical status and description of a particular lesion [2]. Local and multiple AC defects are often approached differently. This depends on the adopted standards of care, surgeon experience and preference, and patient expectations to return to active lifestyle.

Microfracture has been a first-choice treatment for less physically active patients, who are diagnosed with small (<2 cm²) focal chondral or osteochondral defects [3, 4, 5]. However, clinical improvement gradually deteriorates after 2 years, usually because of partial fibrocartilaginous repair tissue, incomplete fill up of the defect and intralesional osteophytes [4, 5]. Additionally, poorer clinical outcome was reported after microfracture of multiple AC defects, when compared to patients with a single defect per knee [6]. OAT has been successfully used for treating small-to medium-sized focal chondral and osteochondral defects of the knee [7]. However, accompanying donor site morbidity prevents its application for larger or multiple defects of the knee. Laboratory culture-based techniques such as MACI have been applied for large, full-thickness, symptomatic AC defects [8, 9, 11]. MACI, and OAT may have clinical benefits over microfracture, superior same level return rate and durability after >3 years [10]. Albeit shown effective in long-term clinical data, a widespread use of MACI procedures is limited by a need for cell manufacturing plant and the cost of the procedure [11].

AMIC has been shown to be clinically safe and effective in isolated, larger (>2 cm²) chondral defect cases [2, 12, 13]. To sustain early mechanical stability and cartilage regeneration, collagen membrane is fixed over the microfracture defect [14]. Initially, indicated defect size for AMIC was 1.5 cm², nevertheless, subsequent studies attempted enlarging a defect area that could be covered with bioactive membrane [14, 15]. Due to increasing number of cases where more than one lesion and thus greater total defect areas are diagnosed, a need for improving treatment of severe cases has become more important recently. Several studies have reported an inferior clinical outcome of greater size lesions [16, 17, 18]. However, some studies did not show influence of defect size on clinical effect [19, 20]. Still, there is scarcity of data about the actual return to previous physical activity levels and clinical outcome after grade I-II defects are diagnosed and left untreated.

Different treatment methods to manage a sustained osteochondral injury have been introduced, however complete restoration of cartilage tissue has not yet been achieved. Recently, scaffold and cellular based tissue engineering have progressed rapidly and currently hold a promise as a useful tool in the field of regenerative orthopedics and especially, cartilage repair.

Similarly, to any medical product, quality parameters for tissue engineered cartilage (TEC) must be established, as well. Safety and efficacy profiles highly depend on characterization of cellular, noncellular components and the interaction in between.

TEC biocompatibility is highly dependent on scaffold biomechanical and physical features in vitro [15, 16]. Customization of scaffolds with highly predictable physical characteristics, such as mechanical rigidity, pore size, porosity and pore interconnectivity are enabled by the ability to adjust parameters of synthetic polymeric feed material [17, 18, 19].

These critical structural parameters provide a homogenous distribution of cells and nutrient transfer within the scaffold [6, 20]. In addition, phenotype formation and superior production of extracellular matrix (ECM) proteins can be influenced by the custom design of pore morphology. Scaffold biomechanics can be similarly affected by the surrounding liquid medium, especially the soft natural collagen-based scaffolds, thus reducing biomechanical properties of the scaffold prior to implantation. The ability of chondrocytes to effectively adhere to the scaffold is highly dependent on cell viability. In addition, viable cell proliferation is indicative of the ability to deposit ECM on the scaffold, thus identification of optimal biomaterial morphological parameters is essential for specific cellular processes. Similarly, TEC potency in vitro is established by cartilaginous protein expression and secretion [16]. A lost ability of monolayer cells to express hyaline cartilage genes could be reversed in a three-dimensional (3D) culture. Therefore, upregulation of genes encoding hyaline cartilage specific type-II collagen is indicative of the cell redifferentiation and functionality of TEC in vitro.

International patent application No. PCT/US2018/064570 (publication No WO2019113511A1) discloses electrospun fibers based scaffolds for repair and regrowth of hyaline cartilage i.e. for treating articular or hyaline cartilage damage or injury using biocompatible electrospun polymer fibers based scaffold. Main disadvantages of the disclosed scaffold are poor biocompatibility, loss of mechanical and tensile strength after application, lack of local anti-acidic mode-of-action, lack of cartilage regeneration supporting properties (for reducing local inflammation).

Polycaprolactone - IUPAC name Poly (hexane-6-lactone) (PCL) is a biodegradable polyester with a low melting point (about 60°C) [21]. This polymer is resistant to mechanical impact. Polycaprolactone is biodegradable and biocompatible. In humans, caprolactone is biodegraded by hydrolysis of ester linkages [2, 3]. D-L poly (glycolic acid), also known as PLG or PLGA (Poly (D, L-lactide-co-glycolide)), is a biodegradable, human-compatible copolymer [22, 23, 24]

However, the synthetic biodegradable polymers have been reported to cause the acidification of the cartilage tissue. They also induced loss of chondrogenic phenotype and the inflammatory responses in vitro [23].

PLGA degradation/ hydrolysis in vivo, causes acidification in the vicinity of the scaffold, thus resulting in the cell toxicity and local inflammation [25]. After the degradation, lactic and glycolic acid are formed as by-products, which in turn negatively affect cell growth, proliferation and tissue regeneration.

Magnesium hydroxide (Mg(OH)₂) is an inorganic compound that is used as a major component of antacids to treat several symptoms such as indigestion or heartburn. It has very low solubility in water, but it can easily dissolve in the presence of acid. Such properties may allow MH to neutralize the acidic degradation product of polymer composites. After neutralization by Mg(OH)₂, abundant magnesium ions are less cytotoxic because of their predominant existence in the body (bone, muscle, and soft tissue). Under in vitro conditions, a high concentration of extracellular calcium ion provokes the plasma membrane damage, thereby leading to cytotoxicity, whereas magnesium ion is reported to be relatively nontoxic. Furthermore, magnesium ions could inhibit the vascular calcification.

Scaffold is considered to be an additional substance for combination with cells and an integral part of the final TEC. It acts as a template to support cell proliferation, interaction, and deposition of ECM. Synthetic scaffolds can be reproducibly manufactured with desired physical characteristics [26, 27, 28].

Determination of scaffold chemical composition is essential to anticipate the biological response. In addition, the synthetic 3D polymer scaffolds with increased mechanical properties are a great foundation for enhancing the target in vitro and in vivo efficacy [23]. However, the synthetic biodegradable polymers have been reported to cause the acidification of the cartilage tissue. They also induced loss of chondrogenic phenotype and the inflammatory responses in vitro. Sufficient physical and biological capacity of the scaffold in vitro enables prediction of the favorable biocompatibility of the TEC to sustain the heavy loads and reduce the inflammation response after implantation in vivo [26].

Zamanlui et al [29] tested PLGA/PCL blend for bone marrow stem cells chondrogenic differentiation. It showed the positive chondrogenic-determined differentiation, however the construct does not address the inflammatory in vivo condition and subsequent scaffold degradation which causes detrimental acidification in the joint. This prevents for quality cartilage regeneration.

PLGA degradation/ hydrolysis in vivo, causes acidification in the vicinity of the scaffold, thus resulting in the cell toxicity and local inflammation [25]. After the degradation, lactic and glycolic acid are formed as by-products, which in turn negatively affect cell growth, proliferation, and tissue regeneration. In addition, chondrocytes dedifferentiate in two-dimensional growth conditions.

The present invention is dedicated to overcoming of the above shortcomings and for producing further advantages over prior art.

### SUMMARY OF INVENTION

Invention is a three-dimensional construct, comprising electrospinned polycaprolactone material scaffold supplemented with polylactic-glycolic acid. The scaffold is augmented with magnesium hydroxide particles, for antiacid properties, which counteract acidifications caused by polymer hydrolysis. In addition, the scaffold is supplemented with chondrocytes establish a qualitative cartilage-specific regeneration in vitro.

The construct is characterized by antiacid and biocompatibility properties for cartilage tissue engineering during the scaffold degradation.

**Technical problem.** PLGA degradation/ hydrolysis in vivo, causes acidification in the vicinity of the scaffold, thus resulting in the cell toxicity and local inflammation. After the degradation, lactic and glycolic acid are formed as by-products, which in turn negatively affect cell growth, proliferation and tissue regeneration. In addition, chondrocytes dedifferentiate in two dimensional growth conditions.

**Solution to problem.** A three-dimensional scaffold, comprised of the electrospinned polycaprolactone (PCL) material supplemented with polylactic-glicolic acid (PLGA). A scaffold is augmented with magnesium hydroxide particles, for antiacid properties, which counteract acidifications caused by polymer hydrolysis. In addition, scaffold supplementation with chondrocytes establish a qualitative cartilage-specific regeneration in vitro.

**Advantageous effects of innovation.** The augmented scaffold is characterized by antiacid and biocompatibility properties for cartilage tissue engineering during the scaffold degradation.

### BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:

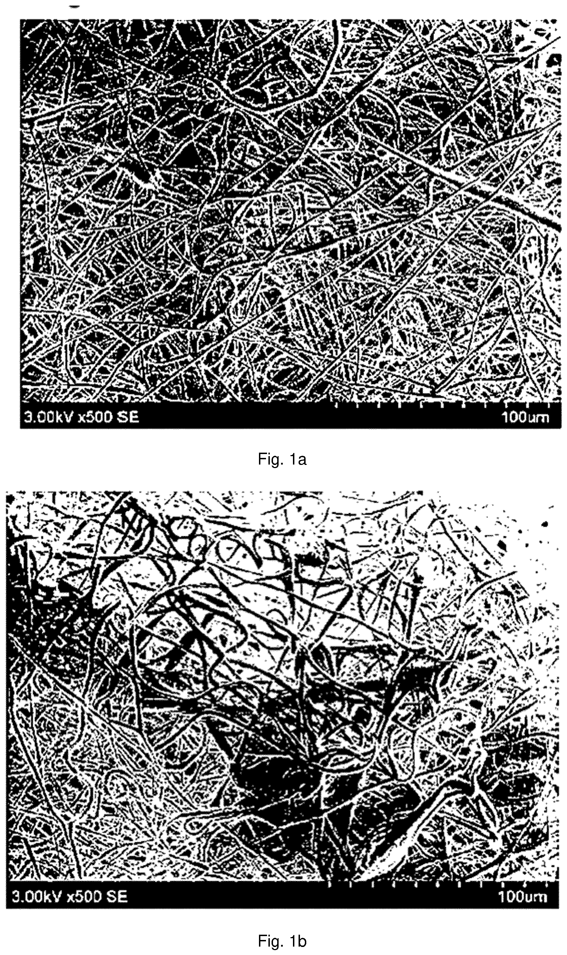

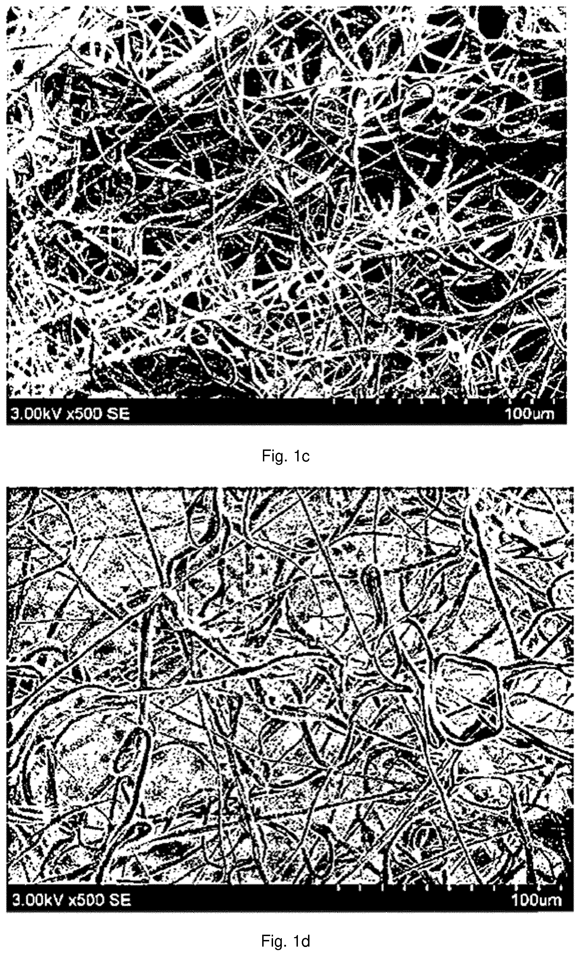

Fig. 1 shows scanned electron microscopy images of examples of scaffolds according to the invention:
   a) PCL:PLGA - 3:1 (wt/wt) - scaffold with added PCL:PLGA, but without added Mg(OH)₂, a control scaffold;
   b) PCL:PLGA - 3:1 (wt/wt) + Mg(OH)₂ 15% - scaffold with added PCL:PLGA and Mg(OH)₂ at 15%;
   c) PCL:PLGA - 3:1 (wt/wt) + Mg(OH)₂ 30% - scaffold with added PCL:PLGA and Mg(OH)₂ at 30%;
   d) PCL:PLGA - 3:1 (wt/wt) + Mg(OH)₂ 45% - scaffold with added PCL:PLGA and Mg(OH)₂ at 45%;
Fig. 2 shows mapping of magnesium (middle) and oxygen (left) elements in: Fig. 2a scaffolds b),
Fig. 2b, scaffold c), and in Fig. 2c scaffold d) of Fig.1, according to the invention
Fig. 3 shows contact angle between the scaffold-water interface in scaffolds: Fig. 3a scaffolds a),
Fig. 3b, scaffold b), in Fig. 3c scaffold c), in Fig. 3d scaffold d) of Fig.1 according to the invention.
Fig. 4. shows FTIR spectra in:
   a) scaffold PCL:PLGA - 3:1 (wt/wt);
   b) PCL:PLGA - 3:1 (wt/wt) + Mg(OH)₂ 15%;
   c) PCL:PLGA - 3:1 (wt/wt) + Mg(OH)₂ 30%;
   d) PCL:PLGA - 3:1 (wt/wt) + Mg(OH)₂ 45%.
Fig. 5 shows pH change dynamics in example of scaffold according to the invention during the scaffold degradation: an untreated P/P/0 scaffold having highest change in pH of the media during 21-day period; Mg(OH)₂ treated P/P/15 and P/P/30 scaffolds exhibit retained physiological pH values. The P/P/30 exhibit the most consistent pH value dynamics during the 21-day period by retaining it in the physiological area. P/P/45 saw the most dramatic initial increase in pH value (acidification), which later subsided however still above the physiological pH value.
Fig. 6 shows biocompatibility level of examples of scaffolds seeded with cells according to the invention. Viability assay revealed the reduced proliferation of chondrocytes with the highest concentration of Mg(OH)₂ in the scaffold material. P/P/30 scaffold provided superior cell proliferation environment and higher chondrocyte proliferation rate in vitro. P/P/45 scaffold was comparable to the untreated scaffold, which was marked by the inferior cell proliferation capabilities in both carriers.
Fig. 7 shows an increased type II collagen gene expression in every, however mostly in P/P/15 and P/P/30 examples of scaffolds throughout the culture period according to the invention. P/P/30 scaffold exhibited the highest expression of type II collagen gene. The P/P/45 revealed a decreased type II collagen gene expression.
Fig. 8 shows superior anti-inflammatory properties of every Mg(OH)₂ treated example of scaffold according to the invention when compared to an untreated scaffold. Treated scaffolds reduced the TNF-alpha expression throughout the culture period, whereas untreated

Preferred embodiments of the invention will be described herein below with reference to the drawings.

### DETAILED DESCRIPTION OF THE INVENTION

It should be understood that numerous specific details are presented to provide a complete and comprehensible description of the invention embodiment. However, the person skilled in art will understand that the embodiment examples do not limit the application of the invention which can be implemented without these specific instructions. Well-known methods, procedures and components have not been described in detail for the embodiment to avoid misleading. Furthermore, this description should not be considered to be constraining the invention to given embodiment examples but only as one of possible implementations of the invention.

Method of producing a bioactive three-dimensional scaffold according to the invention, for reconstruction of symptomatic, local cartilage defects, comprises use of polycaprolactone (PCL) material for making scaffold by electrospinning method,

Method of producing a bioactive three-dimensional scaffold according to the invention, further comprises selecting and using poly-D-L-lactide-co-glycolide (PLGA) with a lactic acid monomer content of 75% in the polymer and a glycolic acid monomer content of 25%, maintaining a monomer ratio of 75:25. This imparts superior biocompatibility properties to the scaffold because of PCL and the retained mechanical and tensile strength because of the PLGA content.

The method according to the invention further comprises addition of Mg(OH)₂ to the scaffold. The Mg(OH)₂ is added at 15-45%, in particular 15%, preferably at 45%, and most preferably at 30%.

The method according to the invention further comprises supplementing the scaffold with chondrogenic cells.

Method of producing bioactive three-dimensional scaffold preferably comprises supplementing the scaffold with combination of Polycaprolactone, PLGA magnesium hydroxide at 30% concentration, and chondrogenic cells.

The PCL and PLGA blend improves biocompatibility. Additional Mg(OH)₂ particles incorporation - overcome the in vivo acidification which is the cause of inflammation. Chondrogenic cells improve articular cartilage repair over regular cell-free scaffold.

The added component of cells, e.g. chondrocytes, greatly improves cartilage regeneration capabilities of the bioactive three-dimensional cellular construct according to the invention for reconstruction of symptomatic, local cartilage defects, as evident from the biocompatibility and type II collagen gene expression studies. Chondrocytes can be used to assess the biocompatibility of the prepared scaffolds. To evaluate the cytocompatibility of the prepared Scaffolds and the viability of the seeded chondrocytes, the Presto Blue viability test can be used. The Presto blue assay is performed at a predetermined time (1, 2, 3, and 4 days).

The manufacturing process of bioactive three-dimensional cellular construct for reconstruction of symptomatic, local cartilage defects consist of:
- scaffold preparation step
   construct preparation steps.,

Examples of scaffolds according to the invention prepared by the method according to the invention:
- PCL/PLGA 3/1 (wt/wt)
- PCL/PLGA 3/1 (wt/wt) + Mg(OH)₂ 15% (of PLGA mass)
- PCL/PLGA 3/1 (wt/wt) + Mg(OH)₂ 30% (of PLGA mass)
- PCL/PLGA 3/1 (wt/wt) + Mg(OH)₂ 45% (of PLGA mass)

Preferably polycaprolactone (PCL) of mol wt 80,000 and PLGA- poly(D,L-lactide-co-glycolide) lactide:glycolide (ratio 75:25), mol wt 66,000-107,000 are used.

Preferably bioactive magnesium hydroxide (Mg(OH)₂ substrate are added in the form of nanopowder, <100 nm.

Preferably acetone and dimetilformamide solvents are used in preparation of polymer solution. Preferably the polymer solution concentration is at 20% wt/v.. Preferably solvent ratio of acetone:dimetilformamide is 2:3 v/v. For example, to create a solution of 10 ml, 4ml of acetone and 6 ml of dimetilformamide 6 ml (ACE/DMF, 2/3, v/v) are used. Polymer is mixed by combining 1,5 g PCL with 0,5 g PLGA, total at 2 g (PCL/PLGA, 3/1, wt/wt) of polymer.

Electrospining parameters for electrospinning a scaffold according to the invention are preferably as follows: polymer solution supply flow 2.3 ml/h; needle no. 21; distance between needle and collector 15 cm; voltage between needle and collector 22 kV; ambient temperature 30°C; relative humidity 40%. Aluminum foil substrate. The needle moves in the x-axis at 5 cm intervals. The polymer solution is supplied with a Teflon hose. As an example, one sample is formed from 10 ml solution. Dry ice is added to the collector every 25 minutes.

Scaffolds after electrospinning are lyophilized by freezing the electrospun scaffolds in a freezer preferably at -20°C for 24 h. Frozen samples are lyophilized under a vacuum of 2 × 10⁻⁶ mPa at -40°C for 72 h.

Preferably, scaffolds after lyophilization are stored in glass airtight vials in the dark at 20-25°C and 40-60% relative humidity.

The morphology of the examples of scaffolds according to the invention can be analyzed, for example, by using scanning electron microscopy, e.g., SEM S-3400N, Hitachi Ltd, Japan. Analysis of magnesium and oxygen atoms can be performed, for example, by electron dispersive X-ray spectroscopy (EDS) using the same equipment as SEM at a voltage of 15 kV and a working distance of 10 mm.

Mechanical properties of examples of scaffolds according to the invention can be determined, for example, by using a universal material testing machine, e.g., BDO-FB 0.5 TH, Zwick GmbH & Co, Germany, in a controlled environment according to ASTM D 882 standard. For example, rectangular samples with a length of 60 mm, a width of 14 mm and a thickness of 0.15 mm can be used for the measurements.

The hydrophilicity of examples of scaffolds according to the invention can be measured, for example, by using a Theta Lite TL 101 optical tensiometer, e.g., Biolin Scientific, Finland).

Chemical changes in examples of scaffolds according to the invention can be analyzed, for example, by using the Fourier Transform Infrared (FTIR) spectra, e.g., Perkin-Elmer Frontier, USA, in the range of 4000 to 650 cm⁻¹.

SEM photographs of the developed and manufactured examples of scaffolds according to the invention are shown in Figures 1-a), 1b), 1c), 1d).. From the SEM photographs it is evident that the layout of the scaffolds consists of a dispersed microfiber structure. In all layouts, the strands are arranged in a random order, the strands are not oriented in one direction.

### Elemental map of magnesium and oxygen, of the examples of the scaffolds according to the invention:

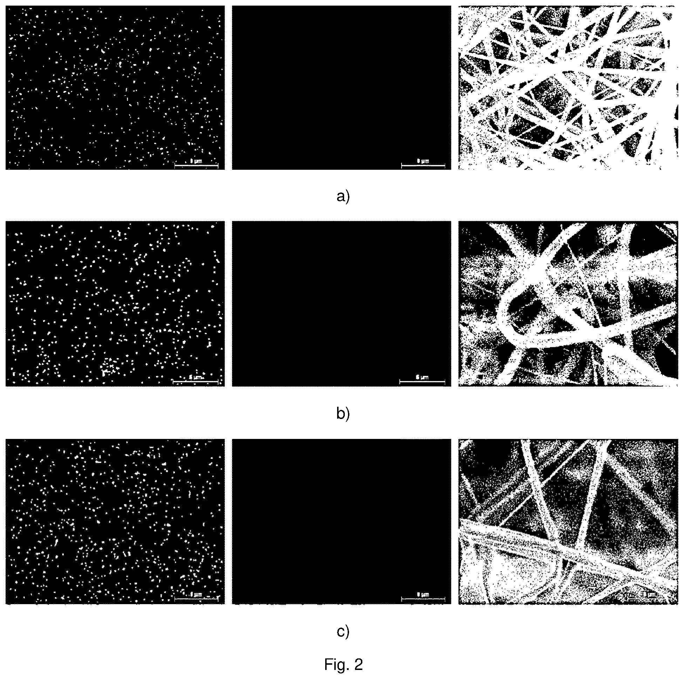

Analysis of Magnesium and oxygen elements in the examples of scaffolds PCL/PLGA 3/1 (wt/wt) + Mg(OH)₂ 15% (of PLGA mass), PCL/PLGA 3/1 (wt/wt) + Mg(OH)₂ 30% (of PLGA mass), PCL/PLGA 3/1 (wt/wt) + Mg(OH)₂ 45% (of PLGA mass) are shown in Fig. 2. The right side shows SEM image of a small portion of the layout, with a map of magnesium atoms in the middle and a map of oxygen atoms on the left. Both magnesium and oxygen atoms in the scaffolds' fibers are evenly distributed, from which it can be concluded that the magnesium hydroxide particles were properly dispersed in the polymer solution and did not adhere to the agglomerates during the production of the scaffolds.

### Mechanical properties of the examples of the scaffolds according to the invention:

Tensile force measurements of the developed and manufactured examples of the scaffolds according to the invention are shown in Table 2. Scaffold No. 1, which consisted only of a mixture of PCL and PLGA 3/1, has a tensile force of 77 ± 6 Pa. The tensile force of the scaffolds with magnesium hydroxide additive ranged from 201 ± 42 Pa to 383 ± 67 Pa. The tensile force of the scaffolds with nicotinic acid additive ranged from 65 ± 7 Pa to 140 ± 28 Pa. Although addition of magnesium hydroxide particles impairs the tensile properties of the scaffolds but improves performance of biological function.

### Hydrophilicity of the examples of the scaffolds according to the invention:

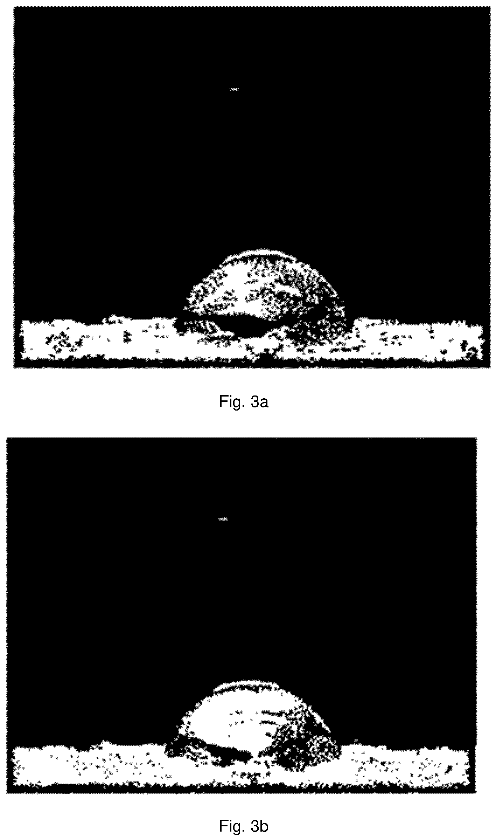

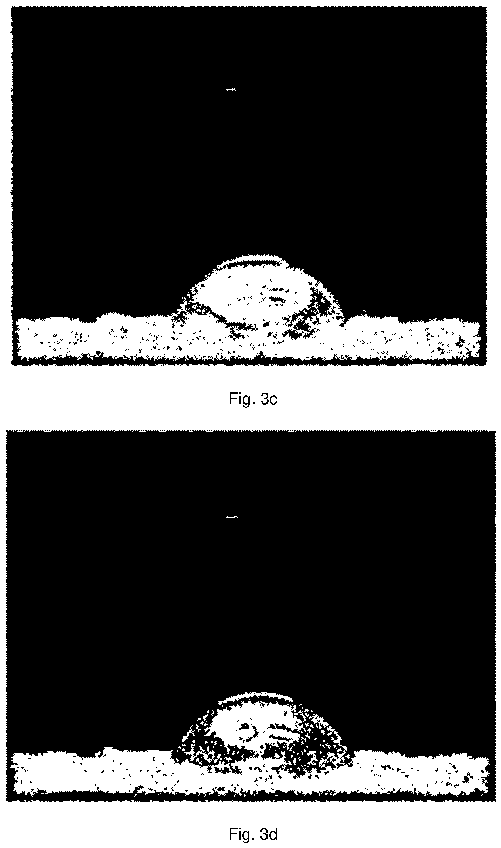

The wetting angle results of the scaffold no1. made of a mixture of PCL and PLGA was 64.7 °. The addition of magnesium hydroxide particles in the scaffolds reduced water wetting angle from 61.3 ° (15% magnesium hydroxide particles) to 53.4 ° (45% particles). All examples of scaffolds according to the invention have hydrophilic properties. The wetting angle results of the scaffold no1. made of a mixture of PCL and PLGA was 64.7 °. Analysis of wetting angles are shown in Fig. 3.

### Functional groups of the surface of layout threads:

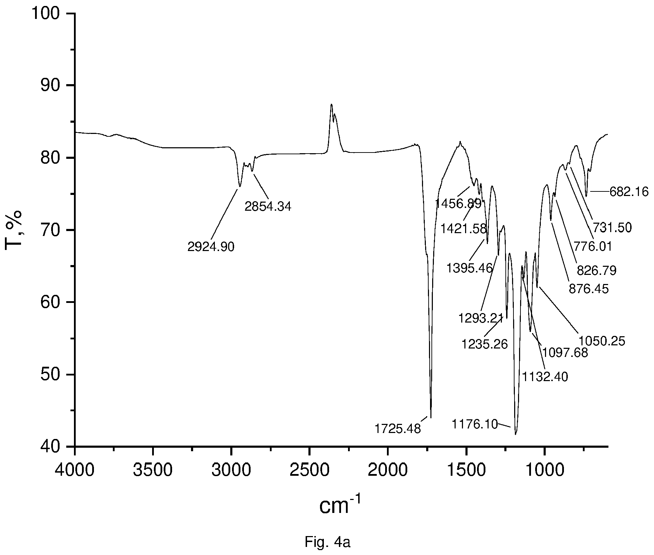

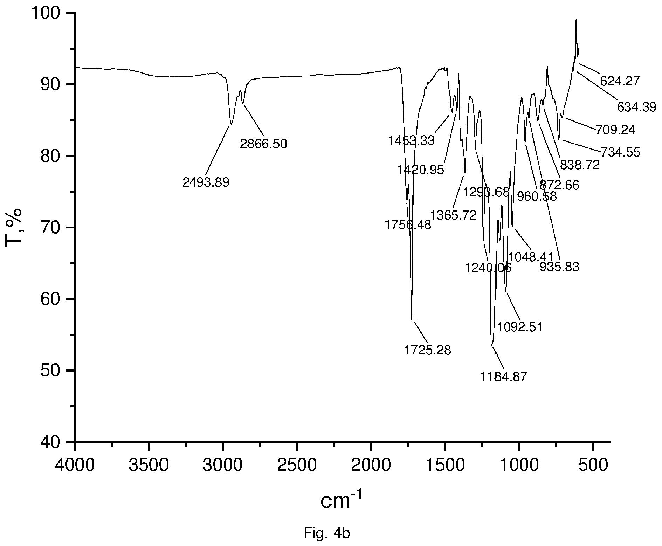

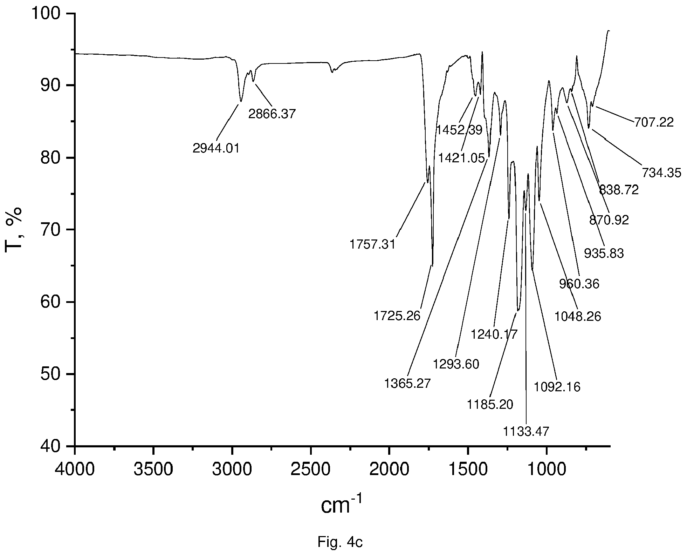

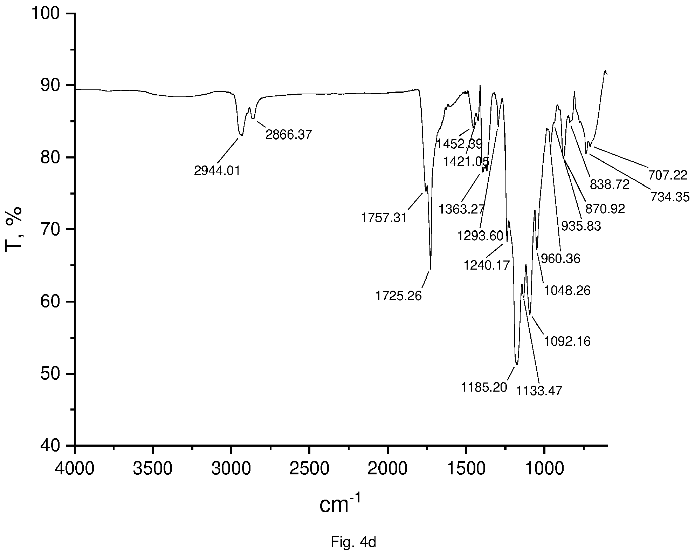

The FTIR spectra of the examples of the scaffolds according to the invention: are presented in Figures 4-a), 4-b), 4-c), 4-d). This analysis allows to determine the presence of functional groups on the surface of the layout threads.

FTIR spectra of all examples of the scaffolds according to the invention have peaks in the range 3000-3700 cm-1, which are characteristic of -OH functional group fluctuations. This peak is broad in nature with low intensity, it is characteristic of the -OH peak in the FTIR spectrum.

### Construct preparation:

In the final step the examples of scaffolds according to the invention were seeded with stem cells. Cell content on one scaffold is restricted to 1×10⁶ cells to 1 cm² area of the scaffold. After trypsinization with Tryple Select, second passage cells are counted and resuspended in vials with culture medium. Cells are seeded in 40 µl doses on presterilized scaffolds in a dropwise fashion. Seeded scaffolds are placed for 2 h in the CO₂ incubator. The seeding of the cells enhances the cartilage restorative properties of the scaffolds, by allowing the cellular adhesion and subsequent proliferation on the scaffolds when in use in therapy. The remaining medium is added to support the effective cell growth and nutrient intake and metabolic waste disposal.

Seeded scaffolds are cultured up to 21 days. Medium is changed every 3-4 days. Scaffolds with cells are harvested after predetermined endpoints for pH, biocompatibility, type II collagen and TNF-α gene expression studies.

At every defined time interval, pH of the media is assessed with a pH meter.

### Biocompatibility testing of examples of scaffolds according to the invention:

All experimental procedures were approved and conducted according to the standard guidelines and protocols set by the Kaunas Regional Biomedical Research Ethics Committee. An informed signed content was obtained from the patient. Human cartilage tissue was collected during routine knee reconstruction surgery procedure as a waste material. Briefly, a biopsy was washed extensively, and digested with collagenase overnight. Isolated cells were plated and cultured in Dulbecco's modified eagle medium supplemented with 10% fetal bovine serum, gentamicin at 37 °C in a humid atmosphere with 5% CO₂. Cells were harvested when reached 80-90% confluence. Scaffolds were soaked in proliferation medium one day before seeding.

Characteristics of scaffolds according to the invention are as follows:
comprises a cylindrical shape, preferably having diameter of 6 mm and a height of 1 mm);
morphological composition of the scaffolds comprises a microfiber structure in which filaments are arranged in a random direction, i.e., non-oriented;
average filament diameter in the scaffolds is preferably in the range from 1×10⁻⁶ to 1×10⁻⁵ m;
statistical dispersion of scaffolds' filament thickness is preferably 90% variance;
scaffolds' filament thread length is preferably 90th percentile value 6×10⁻⁵ m;
scaffolds' porosity is preferably 85%;
mechanical tensile properties of the scaffolds are preferably 0.6 Pa;
hydrophilicity of the scaffolds is preferably wetting angle 70°;
Surface of the scaffolds comprises hydroxyl groups attached thereto.

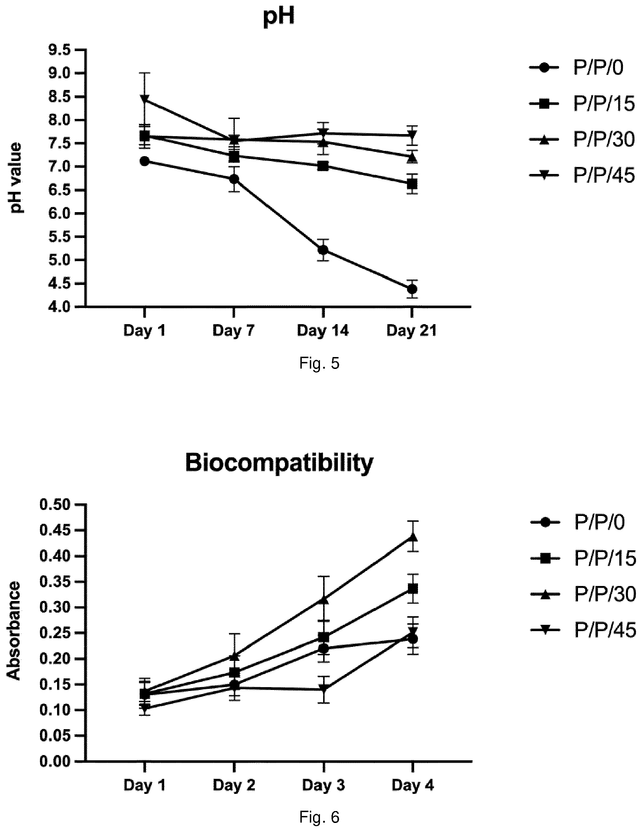

The examples of the scaffolds according to the invention are further characterized by extent of degradation of the scaffolds, pH of untreated scaffold P/P/0, example shown in Fig.5 did not change for the 1^{st} day. Other scaffolds saw an increase in pH values, with respect to the concentration of Mg(OH)₂ used. The initial day 1 saw an acidification of the media, which was later gradually reduced. P/P/45, example shown in Fig.5 saw the most dramatic increase in pH due to the highest Mg(OH)₂ concentration mixed in the scaffold.

The P/P/30, example shown in Fig.5 allowed for the most consistent pH change dynamics during the 21-day period retaining it in the physiological area. Therefore, the sustained physiological pH acidity in the peri-scaffold area might have the most compatible environment for the desirable three-dimensional cell proliferation, and the highest neutralizing effect on the scaffold degradation by-products as shown in Fig. 5, Table 1 and Annex 1 for Table 1.

| **Table 1. pH** | | | | | | | | |
|---|---|---|---|---|---|---|---|---|
| | **P/P/0** | | **P/P/15** | | **P/P/30** | | **P/P/45** | |
| | **Average** | **SD** | **Average** | **SD** | **Average** | **SD** | **Average** | **SD** |
| **Day 1** | 7,12 | 0,10 | 7,67 | 0,20 | 7,65 | 0,25 | 8,43 | 0,58 |
| **Day 7** | 6,73 | 0,27 | 7,23 | 0,14 | 7,58 | 0,46 | 7,55 | 0,12 |
| **Day 14** | 5,22 | 0,23 | 7,02 | 0,08 | 7,53 | 0,27 | 7,72 | 0,23 |
| **Day 21** | 4,38 | 0,19 | 6,63 | 0,21 | 7,22 | 0,13 | 7,67 | 0,21 |
| | | | | | | | | |

| **Annex 1 for Table 1** | |
|---|---|
| **Condition** | **Significant difference** |
| Day 1 :P/P/0 vs. Day 1 :P/P/15 | 0,0352 |
| Day 1 :P/P/0 vs. Day 1 :P/P/30 | 0,0485 |
| Day 1 :P/P/0 vs. Day 1 :P/P/45 | <0,0001 |
| Day 1 :P/P/0 vs. Day 14:P/P/0 | <0,0001 |
| Day 1 :P/P/0 vs. Day 21 :P/P/0 | <0,0001 |
| Day 1 :P/P/15 vs. Day 1 :P/P/45 | 0,0002 |
| Day 1 :P/P/15 vs. Day 14:P/P/15 | 0,0042 |
| Day 1 :P/P/15 vs. Day 21 :P/P/15 | <0,0001 |
| Day 1 :P/P/30 vs. Day 1 :P/P/45 | 0,0002 |
| Day 1 :P/P/45 vs. Day 7:P/P/45 | <0,0001 |
| Day 1 :P/P/45 vs. Day 14:P/P/45 | 0,0008 |
| Day 1 :P/P/45 vs. Day 21 :P/P/15 | <0,0001 |
| Day 1 :P/P/45 vs. Day 21 :P/P/45 | 0,0002 |
| | |
| Day 7:P/P/0 vs. Day 7:P/P/30 | <0,0001 |
| Day 7:P/P/0 vs. Day 7:P/P/45 | <0,0001 |
| Day 7:P/P/0 vs. Day 14:P/P/0 | <0,0001 |
| Day 7:P/P/0 vs. Day 21 :P/P/0 | <0,0001 |
| Day 7:P/P/15 vs. Day 21 :P/P/15 | 0,0127 |
| | |
| Day 14:P/P/0 vs. Day 14:P/P/15 | <0,0001 |
| Day 14:P/P/0 vs. Day 14:P/P/30 | <0,0001 |
| Day 14:P/P/0 vs. Day 14:P/P/45 | <0,0001 |
| Day 14:P/P/0 vs. Day 21 :P/P/0 | <0,0001 |
| Day 14:P/P/15 vs. Day 14:P/P/45 | 0,0013 |
| | |
| Day 21 :P/P/0 vs. Day 21 :P/P/15 | <0,0001 |
| Day 21 :P/P/0 vs. Day 21 :P/P/30 | <0,0001 |
| Day 21 :P/P/0 vs. Day 21 :P/P/45 | <0,0001 |
| Day 21 :P/P/15 vs. Day 21 :P/P/30 | 0,018 |
| Day 21 :P/P/15 vs. Day 21 :P/P/45 | <0,0001 |

### Biocompatibility:

Chondrocytes were used to assess the biocompatibility of the prepared scaffolds. To evaluate the cytocompatibility of the prepared Scaffolds and the viability of the seeded chondrocytes, w Presto Blue viability test was used. 125,000 chondrocytes were suspended in 25 µl of media and seeded on the respective scaffolds in a 6-well plate. Scaffolds with cells were incubated for 60 minutes before the addition of the additional 2 ml of media. The Presto blue assay was performed at a predetermined time (1, 2, 3, and 4 days). The absorbance of the collected Presto blue solution was measured for 5s at 540 nm using a microplate reader with no shaking.

The biocompatibility and cell proliferation on the examples of scaffolds according to the invention were evaluated using human chondrocytes. The *in vitro* Presto blue results demonstrated that the proliferation of chondrocytes is decreased with the higher Mg(OH)₂ concentration.

P/P/30 scaffold provided superior cell proliferation environment by modulating the cytocompatibility within the acidic scaffold. P/P/45 scaffold was comparable to the untreated scaffold, which was marked by the inferior cell proliferation capabilities in both of these carriers.

The scaffold retained and increased the biological scaffold to promote cell survival against the toxicity associated with acidic by-products shown in Fig. 6 and Annex 2 for Table 2 .

| **Table 2. Biocompatibility** | | | | | | | | |
|---|---|---|---|---|---|---|---|---|
| | **P/P/0** | | **P/P/15** | | **P/P/30** | | **P/P/45** | |
| | **Average** | **SD** | **Average** | **SD** | **Average** | **SD** | **Average** | **SD** |
| **Day 1** | 0,13 | 0,03 | 0,13 | 0,02 | 0,14 | 0,03 | 0,10 | 0,01 |
| **Day 7** | 0,15 | 0,02 | 0,17 | 0,03 | 0,21 | 0,04 | 0,14 | 0,02 |
| **Day 14** | 0,22 | 0,03 | 0,24 | 0,03 | 0,32 | 0,04 | 0,14 | 0,03 |
| **Day 21** | 0,24 | 0,03 | 0,34 | 0,03 | 0,44 | 0,03 | 0,25 | 0,03 |

| **Annex 2 for Table 2** | |
|---|---|
| **Condition** | **Significant difference** |
| Day 1 :P/P/0 vs. Day 14:P/P/0 | <0,0001 |
| Day 1:P/P/0 vs. Day 21:P/P/0 | <0,0001 |
| Day 1 :P/P/15 vs. Day 14:P/P/15 | <0,0001 |
| Day 1 :P/P/15 vs. Day 21 :P/P/15 | <0,0001 |
| Day 1:P/P/30 vs. Day 7:P/P/30 | 0,0078 |
| Day 1:P/P/30 vs. Day 14:P/P/30 | <0,0001 |
| Day 1:P/P/30 vs. Day 21 :P/P/30 | <0,0001 |
| Day 1:P/P/45 vs. Day 21 :P/P/45 | <0,0001 |
| | |
| Day 7:P/P/0 vs. Day 14:P/P/0 | 0,0078 |
| Day 7:P/P/0 vs. Day 21 :P/P/0 | 0,0001 |
| Day 7:P/P/15 vs. Day 14:P/P/15 | 0,0107 |
| Day 7:P/P/15 vs. Day 21 :P/P/15 | <0,0001 |
| Day 7:P/P/15 vs. Day 21 :P/P/30 | <0,0001 |
| Day 7:P/P/30 vs. Day 7:P/P/45 | 0,0271 |
| Day 7:P/P/30 vs. Day 14:P/P/30 | <0,0001 |
| Day 7:P/P/30 vs. Day 21 :P/P/30 | <0,0001 |
| Day 7:P/P/45 vs. Day 21 :P/P/45 | <0,0001 |
| | |
| Day 14:P/P/0 vs. Day 14:P/P/30 | <0,0001 |
| Day 14:P/P/0 vs. Day 14:P/P/45 | 0,001 |
| Day 14:P/P/15 vs. Day 14:P/P/30 | 0,0028 |
| Day 14:P/P/15 vs. Day 14:P/P/45 | <0,0001 |
| Day 14:P/P/15 vs. Day 21:P/P/15 | <0,0001 |
| Day 14:P/P/30 vs. Day 14:P/P/45 | <0,0001 |
| Day 14:P/P/30 vs. Day 21 :P/P/30 | <0,0001 |
| Day 14:P/P/45 vs. Day 21 :P/P/45 | <0,0001 |
| | |
| Day 21 :P/P/0 vs. Day 21 :P/P/15 | <0,0001 |
| Day 21 :P/P/0 vs. Day 21 :P/P/30 | <0,0001 |
| Day 21 :P/P/15 vs. Day 21 :P/P/30 | <0,0001 |
| Day 21 :P/P/15 vs. Day 21 :P/P/45 | 0,0003 |
| Day 21 :P/P/30 vs. Day 21 :P/P/45 | <0,0001 |

### Col 2 and TNF alpha expression:

For the chondrogenic gene mRNA analysis, we examined type-II collagen (COL2A1) and TNF alpha mRNAs' expression dynamics of cell seeded scaffolds on days 1, 7, 14, and 21 *in vitro.* For the TNF-α study, we added 10ng/ml of the cytokine to the culture medium for the duration of the study.

The total RNA from the samples was extracted using an ISOLATE II RNA Micro Kit according to the manufacturer's instructions. Elution was performed with 10 µl RNase-free water included in the kit. SensiFAST Probe No-ROX One-Step Kit, primers and hydrolyzation probes were used for One-step RT-qPCR. The primer and probe sequences were designed using Vector NTI Advance^{™} program for sequences alignment and FastPCR online java applet for primers test. All multiplex one-step reactions were carried out in a total volume of 15 µl using 6.75 µl of the isolated RNA sample and primers at a concentration of 200 nM each, and probes at a concentration of 100 nM each. The multiplex one-step RT-qPCR assays were performed employing a real-time thermocycler Rotor-Gene Q 5plex model with software version 1.7 under the following conditions: first strand cDNA was synthesized at 45 °C for 20 min (1 cycle) and then denatured at 95 °C for 2 min (1 cycle); followed by 50 cycles of denaturation at 95 °C for 10 s and annealing/extension at 60 °C for 1 min. The expression of β-actin mRNA was used as an internal standard for normalization of the target mRNA levels between different samples. The 2^-ddCT method was applied for the relative gene expression data evaluation. *Oryctolagus cuniculus* GAPDH gene expression was used for the data normalization.

### Result (Col2):

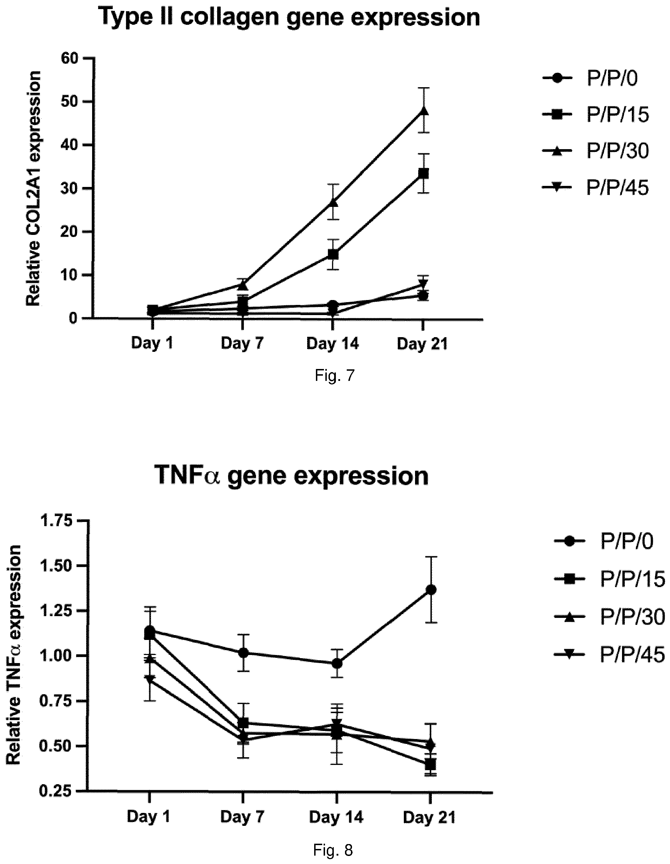

Type II collagen expression was increased in all scaffolds throughout the culture period, however P/P/30 scaffold exhibited the highest proliferation of ECM rate, as evident by the collagen protein expression. The scaffold with the highest content of the Mg(OH)₂ revealed a disturbed type II collagen expression, thus revealing a detrimental effect on the cell biocompatibility when using the highest Mg(OH)₂ concentration shown in Fig. 7, Table 3 and Annex 3 for Table 3.

| **Table 3. Type II collagen expression** | | | | | | | | |
|---|---|---|---|---|---|---|---|---|
| | **P/P/0** | | **P/P/15** | | **P/P/30** | | **P/P/45** | |
| | **Average** | **SD** | **Average** | **SD** | **Average** | **SD** | **Average** | **SD** |
| **Day 1** | 1,63 | 0,67 | 2,02 | 0,60 | 1,97 | 0,73 | 1,23 | 0,23 |
| **Day 7** | 2,43 | 0,46 | 4,03 | 1,51 | 8,00 | 1,26 | 1,25 | 0,34 |
| **Day 14** | 3,33 | 0,37 | 15,00 | 3,46 | 27,17 | 4,07 | 1,33 | 0,31 |
| **Day 21** | 5,58 | 1,12 | 33,78 | 4,48 | 48,35 | 5,20 | 8,13 | 2,05 |

| **Annex 3 for Table 3** | |
|---|---|
| **Condition** | **Significant difference** |
| Day 1 :P/P/15 vs. Day 14:P/P/15 | <0,0001 |
| Day 1:P/P/15 vs. Day 21 :P/P/15 | <0,0001 |
| Day 1:P/P/30 vs. Day 7:P/P/30 | 0,0024 |
| Day 1:P/P/30 vs. Day 14:P/P/30 | <0,0001 |
| Day 1:P/P/30 vs. Day 21 :P/P/30 | <0,0001 |
| Day 1:P/P/45 vs. Day 21 :P/P/45 | 0,0002 |
| | |
| Day 7:P/P/0 vs. Day 7:P/P/30 | 0,0079 |
| Day 7:P/P/15 vs. Day 14:P/P/15 | <0,0001 |
| Day 7:P/P/15 vs. Day 21 :P/P/15 | <0,0001 |
| Day 7:P/P/30 vs. Day 7:P/P/45 | 0,0003 |
| Day 7:P/P/30 vs. Day 14:P/P/30 | <0,0001 |
| Day 7:P/P/30 vs. Day 21 :P/P/30 | <0,0001 |
| Day 7:P/P/45 vs. Day 21 :P/P/45 | 0,0002 |
| | |
| Day 14:P/P/0 vs. Day 14:P/P/15 | <0,0001 |
| Day 14:P/P/0 vs. Day 14:P/P/30 | <0,0001 |
| Day 14:P/P/15 vs. Day 14:P/P/30 | <0,0001 |
| Day 14:P/P/15 vs. Day 14:P/P/45 | <0,0001 |
| Day 14:P/P/15 vs. Day 21 :P/P/15 | <0,0001 |
| Day 14:P/P/30 vs. Day 14:P/P/45 | <0,0001 |
| Day 14:P/P/30 vs. Day 21 :P/P/30 | <0,0001 |
| Day 14:P/P/45 vs. Day 21 :P/P/45 | 0,0003 |
| | |
| Day 21 :P/P/0 vs. Day 21 :P/P/15 | <0,0001 |
| Day 21 :P/P/0 vs. Day 21 :P/P/30 | <0,0001 |
| Day 21 :P/P/15 vs. Day 21 :P/P/30 | <0,0001 |
| Day 21 :P/P/15 vs. Day 21 :P/P/45 | <0,0001 |
| Day 21 :P/P/30 vs. Day 21 :P/P/45 | <0,0001 |

### Result (TNF-a):

The relationship between the scaffold degradation process and the subsequent inflammatory processes, as revealed by the TNF-alpha expression was evaluated during the chondrocyte culture. All scaffolds with incorporated Mg(OH)₂ revealed superior anti-inflammatory properties when compared to the untreated scaffolds. The TNF-alpha expression has seen a continuous decline in the P/P/15 and P/P/30 scaffolds. The slight increase of TNF-α expression in the P/P/45 at the day14 was also evident, revealing a change in the antacid properties of the scaffold throughout the period as shown in Fig. 8, Table 4 and Annex 4 for Table 4.

| **Table 4. TNF-a expression** | | | | | | | | |
|---|---|---|---|---|---|---|---|---|
| | **P/P/0** | | **P/P/15** | | **P/P/30** | | **P/P/45** | |
| | **Average** | **SD** | **Average** | **SD** | **Average** | **SD** | **Average** | **SD** |
| **Day 1** | 1,14 | 0,13 | 1,12 | 0,13 | 0,99 | 0,11 | 0,87 | 0,11 |
| **Day 7** | 1,02 | 0,10 | 0,63 | 0,11 | 0,58 | 0,06 | 0,54 | 0,10 |
| **Day 14** | 0,96 | 0,08 | 0,59 | 0,12 | 0,57 | 0,17 | 0,63 | 0,07 |
| **Day 21** | 1,38 | 0,18 | 0,40 | 0,06 | 0,53 | 0,10 | 0,49 | 0,14 |

| **Annex 4 for Table 4** | |
|---|---|
| **Condition** | **Significant difference** |
| Day 1 :P/P/0 vs. Day 1 :P/P/45 | 0,0076 |
| Day 1 :P/P/0 vs. Day 7:P/P/0 | 0,8999 |
| Day 1 :P/P/15 vs. Day 1 :P/P/45 | 0,0215 |
| Day 1 :P/P/15 vs. Day 7:P/P/15 | <0,0001 |
| Day 1 :P/P/15 vs. Day 14:P/P/15 | <0,0001 |
| Day 1 :P/P/15 vs. Day 21 :P/P/15 | <0,0001 |
| Day 1 :P/P/30 vs. Day 7:P/P/30 | <0,0001 |
| Day 1 :P/P/30 vs. Day 14:P/P/30 | <0,0001 |
| Day 1 :P/P/30 vs. Day 21 :P/P/30 | <0,0001 |
| Day 1 :P/P/45 vs. Day 7:P/P/45 | 0,0005 |
| Day 1 :P/P/45 vs. Day 14:P/P/45 | 0,0451 |
| Day 1 :P/P/45 vs. Day 21 :P/P/45 | <0,0001 |
| | |
| Day 7:P/P/0 vs. Day 7:P/P/15 | <0,0001 |
| Day 7:P/P/0 vs. Day 7:P/P/30 | <0,0001 |
| Day 7:P/P/0 vs. Day 7:P/P/45 | <0,0001 |
| Day 7:P/P/0 vs. Day 21 :P/P/0 | <0,0001 |
| | |
| Day 14:P/P/0 vs. Day 14:P/P/15 | <0,0001 |
| Day 14:P/P/0 vs. Day 14:P/P/30 | <0,0001 |
| Day 14:P/P/0 vs. Day 14:P/P/45 | 0,0003 |
| Day 14:P/P/0 vs. Day 21 :P/P/0 | <0,0001 |
| | |
| Day 21 :P/P/0 vs. Day 21 :P/P/15 | <0,0001 |
| Day 21 :P/P/0 vs. Day 21 :P/P/30 | <0,0001 |
| Day 21 :P/P/0 vs. Day 21 :P/P/45 | <0,0001 |

### NON-PATENT LITERATURE

1. Curl WW, Krome J, Gordon ES, et al. Cartilage injuries: a review of 31,516 knee arthroscopies. Arthroscopy 1997; 13(4): 456-460.
2. Vannini F, Battaglia M, Buda R, et al. "One step" treatment of juvenile osteochondritis dissecans in the knee: clinical results and T2 mapping characterisation. Orthop Clin North Am 2012; 43(2): 237-244.
3. Steadman JR, Briggs KK, Rodrigo JJ, et al. Outcomes of microfracture for traumatic chondral defects of the knee: average 11-year follow-up. Arthroscopy 2003; 19: 477-484.
4. Steinwachs MR, Guggi T, and Kreuz PC. Marrow stimulation techniques. Injury 2008; 39(Suppl 1): S26-S31.
5. Kreuz PC, Steinwachs MR, Erggelet C, et al. Results after microfracture of full-thickness chondral defects in different compartments in the knee. Osteoarthritis Cartilage 2006; 14(11): 1119-1125.
6. Solheim E, Oyen J, Hegna J, et al. Microfracture treatment of single or multiple articular cartilage defects of the knee: a 5-year median follow-up of 110 patients. Knee Surg Sports Traumatol Arthrosc 2010; 18(4): 504-508.
7. Hangody L and Fu" les P. Autologous osteochondral mosaicplasty for the treatment of full-thickness defects of weight-bearing joints: ten years of experimental and clinical experience. J Bone Joint Surg Am 2003; 85-A(Suppl 2): 25-32.
8. Brittberg M, Lindahl A, Nilsson A, et al. Treatment of deep cartilage defects in the knee with autologous chondrocyte transplantation. N Engl J Med 1994; 331: 889-895.
9. Willers C, Chen J, Wood D, et al. Autologous chondrocyte implantation with collagen bioscaffold for the treatment of osteochondral defects in rabbits. Tissue Eng 2005; 11: 1065-1076.
10. Mithoefer K and Della Villa S. Return to sports after articular cartilage repair in the football (soccer) player. Cartilage 2012; 3(1 Suppl): 57S 62S.
11. Peterson L, Minas T, Brittberg M, et al. Treatment of osteochondritis dissecans of the knee with autologous chondrocyte transplantation: results at two to ten years. J Bone Joint Surg Am 2003; 85-A(Suppl 2): 17-24.
12. Gille J, Behrens P, Volpi P, et al. Outcomes of autologous matrix induced chondrogenesis (AMIC) in cartilage knee surgery: data of the AMIC registry. Arch Orthop Trauma Surg 2013; 133(1): 87-93.
13. Kusano T, Jakob RP, Gautier E, et al. Treatment of isolated chondral and osteochondral defects in the knee by autologous matrix-induced chondrogenesis (AMIC). Knee Surg Sports Traumatol Arthrosc 2012; 20(10): 2109-2115.
14. Benthien JP and Behrens P. Autologous matrix-induced chondrogenesis (AMIC). A one-step procedure for retropatellar articular resurfacing. Acta Orthop Belg 2010; 76(2): 260-263.
15. Anders S, Volz M, Frick H, et al. A Randomized, controlled trial comparing autologous matrix-induced chondrogenesis (AMIC®) to microfracture: analysis of 1- and 2-year follow-up data of 2 centers. Open Orthop J 2013; 7: 133-143.
16. Gudas R, Kalesinskas RJ, Kimtys V, et al. A prospective randomized clinical study of mosaic osteochondral autologous transplant versus microfracture for the treatment of osteochondral defects in the knee joint in young athletes. Arthroscopy 2005; 21: 1066-1075.
17. Kim YS, Choi YJ, and Koh YG. Mesenchymal stem cell implantation in knee osteoarthritis: an assessment of the factors influencing clinical outcomes. Am J Sports Med 2015; 43(9): 2293-2301.
18. Knutsen G, Engebretsen L, Ludvigsen TC, et al. Autologous chondrocyte implantation compared with microfracture in the knee: a randomized trial. J Bone Joint Surg Am 2004; 86: 455-464.
19. Behery OA, Harris JD, Karnes JM, et al. Factors influencing the outcome of autologous chondrocyte implantation: a systematic review. Knee Surg 2013; 26(3): 203-211.
20. Pestka JM, Feucht MJ, Porichis S, et al. Return to sports activity and work after autologous chondrocyte implantation of the knee: which factors influence outcomes? Am J Sports Med 2016; 44(2): 370-377.
21. L. Averous, L. Moro, P. Dole, C. Fringant, Properties of thermoplastic blends: starch-polycaprolactone, Polymer (Guildf). 41 (2000) 4157-4167. https://doi.org/10.1016/S00323861(99)00636-9.
22. H.K. Makadia, S.J. Siegel, Poly Lactic-co-Glycolic Acid (PLGA) as Biodegradable Controlled Drug Delivery Carrier, Polymers (Basel). 3 (2011) 1377.
23. H. Song, Y. Kim, J. Park, M. Park, S. Lyu, Y. Koh, J. Heo, D. Lee, K. Park, Biocompatible nanoparticle PLGA is a noble safe delivery system for embryo development and next generations, Fertil. Steril. 108 (2017) e154.
24. K. Park, S. Skidmore, J. Hadar, J. Garner, H. Park, A. Otte, B.K. Soh, G. Yoon, D. Yu, Y. Yun, B.K. Lee, X. Jiang, Y. Wang, Injectable, long-acting PLGA formulations: Analyzing PLGA and understanding microparticle formation, J. Control. Release. 304 (2019) 125-134. https://doi.orq/10.1016/J.JCONREL.2019.05.003.
25. Houchin M.; Topp E. Chemical degradation of peptides and proteins in PLGA: a review of reactions and mechanisms. J. Pharm. Sci. 2008, 97 (7), 2395.
26. B. Dhandayuthapani, Y. Yoshida, T. Maekawa, D. Sakthi Kumar, Polymericscaffolds in tissue engineering application, a review, Int. J. Polym. Sci.290602 (2011) 1-19.
27. J. Zhu, R.E. Marchant, Design properties of hydrogel tissue-engineeringscaffolds, Expert Rev. Med. Devices 8 (2011) 607-626
28. Nikolova MP, Chavali MS. Recent advances in biomaterials for 3D scaffolds: A review. Bioact Mater. 2019;4:271-292. Published 2019 Oct 25
29. Soheila Zamanlui, Matin Mahmoudifard, Masoud Soleimani, Behnaz Bakhshandeh, Mohammad Vasei & Shahab Faghihi (2018) Enhanced chondrogenic differentiation of human bone marrow mesenchymal stem cells on PCL/PLGA electrospun with different alignments and compositions, International Journal of Polymeric Materials and Polymeric Biomaterials, 67:1, 50-60

## Claims

1. Method for producing an electrospinned three-dimensional scaffold for cartilage regeneration, comprising electrospinning a scaffold **characterized by**
- use of polycaprolactone (PCL) material for making scaffold by electrospinning;
- selecting and using poly-D-L-lactide-co-glycolide (PLGA) with a lactic acid monomer content;
- addition of Mg(OH)₂ to the scaffold;
wherein acetone and dimetilformamide solvents are used in preparation of polymer solution;
- lyophilizing by freezing the electrospun scaffold in a freezer preferably at -20°C for 24 h;
- lyophilizing frozen samples under a vacuum of 2 × 10⁻⁶ mPa at -40°C for 72 h;
- storing scaffold after lyophilization in glass airtight vials in the dark at 20-25°C and 40-60% relative humidity,
- adding chondrocytes.

2. Method according to claim 1, where poly-D-L-lactide-co-glycolide (PLGA) with a lactic acid monomer content is of 75% in the polymer and a glycolic acid monomer content of 25%, maintaining a monomer ratio of 75:25.

3. Method according to claim 1 or 2 wherein the Mg(OH)₂ is added at 15-45%, in particular 15%, preferably at 45%, and most preferably at 30%.

4. Method according to any one claims of 1 - 3, wherein polycaprolactone (PCL) of mol wt 80,000 and PLGA- Poly(D,L-lactide-co-glycolide) lactide:glycolide (ratio 75:25), mol wt 66,000-107,000 are used.

5. Method according to any one claims of 1 - 4, wherein the bioactive magnesium hydroxide (Mg(OH)₂) substrate is added in the form of nanopowder, <100 nm.

6. Method according to any one claims of 1 - 5, wherein acetone and dimetilformamide solvents are used in preparation of polymer solution having concentration at 20% wt/v..

7. Method according to any one claims of 1 - 6, wherein solvent ratio of acetone:dimetilformamide is 2:3 v/v.

8. Method according to any one claims of 1 - 7, wherein electrospining parameters for electrospinning a scaffold according to the invention are as follows:
- polymer solution supply flow 2.3 ml/h;
- needle no. 21;
- distance between needle and collector 15 cm;
- voltage between needle and collector 22 kV;
- ambient temperature 30°C;
- relative humidity 40%;
- using Aluminum foil substrate;
- the needle moves in the x-axis at 5 cm intervals;
- the polymer solution is supplied with a Teflon hose;
- adding drying ice to the collector every 25 minutes.

9. A bioactive three-dimensional construct for cartilage regeneration, produced by method according to any one of claims 1-9, comprising electrospun filaments, **characterized in that** it comprises polycaprolactone, poly-D-L-lactide-co-glycolide, Mg(OH)₂, and cartilage tissue cells seeded onto the scaffold.

10. Bioactive three-dimensional construct according to claim 9, where the scaffold comprises PCL/PLGA 3/1 (wt/wt) + Mg(OH)₂ 15% (of PLGA mass).

11. Bioactive three-dimensional construct according to claim 9, where the scaffold comprises PCUPLGA 3/1 (wt/wt) + Mg(OH)₂ 30% (of PLGA mass).

12. Bioactive three-dimensional construct according to claim 9, where the scaffold comprises PCUPLGA 3/1 (wt/wt) + Mg(OH)₂ 45% (of PLGA mass).

13. Bioactive three-dimensional cellular construct according to any one claim 9-12, wherein the cartilage tissue cells are seeded at 1×10⁶ cells to 1 cm² area of scaffold.

14. Bioactive three-dimensional cellular construct according to any one claim 9-13, wherein the seeded scaffold is cultured for up to 21 days.
